# EUROPÄISCHE PATENTSCHRIFT

(11) **EP 2 675 407 B1**
(45) Veröffentlichungstag und Bekanntmachung des Hinweises auf die Patenterteilung: **23.09.2015**
(21) Anmeldenummer: 12717165.0
(22) Anmeldetag: 14.02.2012
(51) Int. Cl.: A61F 9/007, A61B 17/34

(54) **VORRICHTUNG ZUM EINLEITEN VON LICHT IN EIN MENSCHLICHES ODER TIERISCHES AUGE**
DEVICE FOR INTRODUCING LIGHT INTO A HUMAN OR ANIMAL EYE
DISPOSITIF DESTINÉ À ENVOYER DE LA LUMIÈRE DANS UN OEIL HUMAIN OU ANIMAL

(30) Priorität: 14.02.2011 DE 102011011192
(43) Veröffentlichungstag der Anmeldung: 25.12.2013
(73) Patentinhaber: Geuder AG, 69126 Heidelberg (DE)
(72) Erfinder: DRAHEIM, René, 69207 Sandhausen (DE); FRAUENFELD, Dieter, 69121 Heidelberg (DE); GEUDER, Volker, 69126 Heidelberg (DE)
(74) Vertreter: Patent- und Rechtsanwälte Ullrich & Naumann
(86) Internationale Anmeldenummer: PCT/DE2012/200009
(87) Internationale Veröffentlichungsnummer: WO 2012/110034

(56) Entgegenhaltungen:
- EP-A1- 2 392 274
- WO-A1-2007/133267
- DE-A1-102009 032 184
- US-A- 4 744 360
- US-A- 5 725 514
- US-A1- 2003 169 603
- US-B1- 7 258 694

## Beschreibung

Die Erfindung betrifft eine Vorrichtung zum Einleiten von Licht in ein menschliches oder tierisches Auge, mit einem Lichtleiter, der einen Anschlussbereich zum Ankoppeln an eine Lichtquelle und einen am freien Ende ausgebildeten Wirkbereich zum Abstrahlen von Licht aufweist.

An dieser Stelle sei angemerkt, dass es sich hierbei um Licht im weitesten Sinne handelt. Dies kann für den Menschen sichtbares Licht zum Beleuchten der Stelle eines chirurgischen Eingriffs sein. Des Weiteren kann es sich hierbei beispielsweise auch um Infrarot- oder Ultraviolett-Strahlung handeln.

Bei chirurgischen Eingriffen am menschlichen oder tierischen Auge besteht aus verschiedenen Gründen die Notwendigkeit Licht in das Augeninnere einzubringen. Dazu wird bisher zunächst mit einem Skalpell oder einem lanzenartigen Instrument ein Schnitt (Inzision) in der Sklera angebracht. Durch diese Inzision wird ein sogenannter Beleuchtungstrokar in das Augeninnere eingeführt. Der Beleuchtungstrokar dient als Zugang für eine Beleuchtungseinrichtung zum Einführen von Licht in das Auge des Patienten. Durch den Beleuchtungstrokar werden die Wundränder geschont und es kann eine gewisse Abdichtung des Augeninneren gegen die Außenumgebung erreicht werden.

Problematisch ist hierbei, dass nach dem Einsetzen des Beleuchtungstrokars die Gefahr besteht, dass dieser von dem Operateur aus Unachtsamkeit versehentlich berührt und dadurch aus dem Auge herausgezogen wird. Hierbei kann es zu erheblichen Verletzungen, insbesondere an den Wundrändern der Inzision kommen. Ein weiteres Problem liegt darin, dass durch die verschiedenen Arbeitsschritte und die dazu benötigten unterschiedlichen Instrumente das Einbringen von Licht in das Augeninnere äußerst zeitaufwändig und kompliziert ist. Des Weiteren ist problematisch, dass der Beleuchtungstrokar aufgrund des erhöhten intraokularen Ducks nach außen abdichten muss, um ein Kollabieren der Augeninnenwand zu verhindern. Hierzu sind in der Praxis Membrane in dem Durchgangskanal der Beleuchtungstrokare vorgesehen. Dies erhöht die Fertigungskosten eines Beleuchtungstrokars in nicht unerheblichem Maße. Bei einem Beleuchtungstrokar handelt es sich um einen Wegwerfartikel, so dass sich dies auf die apparativen Kosten spürbar auswirkt. Eine entsprechende Abdichtung des Augeninneren gegenüber der Umgebung ist trotz einer Membrane im Durchgangskanal des Beleuchtungskörpers nicht immer gegeben. Ein Abfall des intraokularen Drucks kann jedoch zu irreparablen Verletzungen des Auges führen.

Eine Vorrichtung gemäß dem Oberbegriff des Anspruchs 1 lehrt die WO 2007/133267.

Der vorliegenden Erfindung liegt daher die Aufgabe zugrunde, eine gattungsbildende Vorrichtung zum Einleiten von Licht in ein menschliches oder tierisches Auge derart auszugestalten und weiterzubilden, dass sie sich bei geringstmöglichem Aufwand sicher in das menschliche oder tierische Auge einsetzen lässt, wobei das Auftreten von Komplikationen minimiert sein soll. Die apparativen Kosten sollen so gering wie möglich sein.

Die voranstehende Aufgabe wird erfindungsgemäß durch eine Vorrichtung mit den Merkmalen des Patentanspruchs 1 gelöst. Danach ist die gattungsbildende Vorrichtung dadurch gekennzeichnet, dass der Wirkbereich zumindest teilweise als Stich- bzw. Schneidwerkzeug ausgebildet ist, so dass das freie Ende ohne weitere Hilfsmittel in das Augeninnere einbringbar ist.

Erfindungsgemäß ist erkannt worden, dass die zugrundeliegende Aufgabe durch eine geschickte Ausgestaltung des Wirkbereichs in überraschend einfacher Weise gelöst werden kann. Hierzu ist der Wirkbereich zumindest teilweise als Stich- bzw. Schneidwerkzeug ausgebildet. Durch diese einfache konstruktive Maßnahme ist es dem Operateur nunmehr möglich, die notwendige Inzision an der Sklera durch die Beleuchtungsvorrichtung selbst vorzunehmen. Sowohl die Verwendung eines Schneidinstruments als auch eines Beleuchtungstrokars ist dadurch nicht mehr notwendig. Vielmehr wird die Inzision in der Sklera durch den als Stich- bzw. Schneidwerkzeug ausgebildeten Wirkbereich angebracht und das freie Ende des Lichtleiters direkt bis zur gewünschten Stelle in das Augeninnere eingeführt. Da zum Einleiten von Licht in das Auge nunmehr nur noch eine einzige, konstruktiv einfache Vorrichtung nötig ist, verringert sich die Dauer des Eingriffs erheblich. Des Weiteren werden die Inzisionsränder in weitaus geringerem Maße beansprucht, da nur einmal ein Objekt durch die Sklera hindurch gestoßen werden muss. Während die Inzision gesetzt wird, kann nunmehr auch auf eine weitere Beleuchtung verzichtet werden, da die Inzisionsstelle durch den Wirkbereich ausreichend beleuchtet ist. Somit kann ein derartiger chirurgischer Eingriff mit einem Minimum an Instrumenten durchgeführt werden.

In vorteilhafter Weise ist der Wirkbereich spitz und/oder zumindest bereichsweise leicht gerundet ausgebildet. Wesentlich ist hierbei, dass der Wirkbereich das Durchstoßen der Sklera mit geringem Kraftaufwand ermöglicht.

Der Wirkbereich kann des Weiteren einen Gewindegang oder eine gewendelte Korkenzieherform aufweisen. Jedwede Geometrie, die ein einfaches Einbringen des freien Endes in das Augeninnere ermöglicht, ist hierbei denkbar. Insbesondere ist es von Vorteil, wenn der Wirkbereich scharfe Schneidkanten bzw. Vorsprünge aufweist. Somit ist es dem Operateur möglich, die Sklera nicht nur zu durchstechen, sondern einen Schnitt beliebiger Länge anzubringen.

Um den Abstrahlwinkel des Lichts beeinflussen zu können, kann der Wirkbereich zumindest teilweise beschichtet oder ummantelt sein. Hierdurch ist der Bereich, in dem das Licht abgestrahlt wird exakt definierbar.

In weiter vorteilhafter Weise weist der Wirkbereich eine derartige Geometrie auf, dass das Licht auf einen Punkt fokussiert abstrahlbar ist. Im Konkreten entspricht dies einer Spotbeleuchtung. Des Weiteren ist denkbar, dass die Geometrie des Wirkbereichs derart ausgebildet ist, dass das Licht in einem weiten Winkel abstrahlbar ist. Generell kann der Wirkbereich in Form einer Linse ausgeführt sein, so dass das Licht in einem beliebigen Winkelbereich abstrahlbar ist.

Zur sicheren Verbindung des freien Endes mit dem Auge ist in vorteilhafter Weise ein Anschlusskörper vorgesehen, der das freie Ende begrenzt und möglichst eng bzw. dicht an der Oberfläche des Auges angelegt wird. Hierbei ist es von besonderem Vorteil, wenn der Anschlusskörper flach, insbesondere scheibenartig, tellerartig oder knopfartig, ausgebildet ist. Durch die flache Ausgestaltung des Anschlusskörpers bietet dieser so gut wie keine Angriffsfläche gegenüber anderen Instrumenten oder gegenüber dem Operateur, so dass ein ungewolltes Herausstreifen des freien Endes nahezu ausgeschlossen ist.

Der Anschlusskörper kann als integraler Bestandteil des Lichtleiters vorgesehen sein. Des Weiteren kann der Anschlusskörper als separates Bauteil gefertigt und zur sicheren Befestigung mit dem Lichtleiter an diesen angeklemmt, angepresst oder angeklebt sein. Jegliche mechanische oder adhäsive Verbindung ist hierbei denkbar.

In besonders vorteilhafter Weise ist der Anschlusskörper zumindest bereichsweise oder insgesamt durchsichtig ausgeführt. Hierdurch wird insbesondere während des Setzens der Inzision die Sicht für den Operateur verbessert.

In Bezug auf eine sichere Fixierung der Vorrichtung bzw. des freien Endes im Auge ist es von weiterem Vorteil, wenn das freie Ende zumindest in einem an den Anschlusskörper angrenzenden Fixierungsbereich eine raue, genoppte oder im Wesentlichen quer oder schräg zur Längsachse gewellte oder geriefte Oberfläche aufweist. Alternativ ist es denkbar, dass der Fixierungsbereich durch ein Außengewinde des freien Endes gebildet ist. Des Weiteren kann das freie Ende als Drehteil ausgeführt und der Fixierungsbereich durch Drehriefen gebildet sein.

Der Lichtleiter kann in vorteilhafter Weise aus Glasfaser gefertigt sein. Hierbei kann es sich beispielsweise um Mineralglas bzw. Kieselglas handeln. Des Weiteren ist denkbar, den Lichtleiter aus polymeren optischen Fasern besteht, wobei es sich hierbei insbesondere um Polymethylmethacrylat (PMMA) handeln kann.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung von vier bevorzugten Ausführungsbeispielen der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein erstes Ausführungsbeispiel der erfindungsgemäßen Vorrichtung,
- Fig. 2: in einer schematischen Ansicht, vergrößert, den Wirkbereich eines ersten Ausführungsbeispiels einer erfindungsgemäßen Vorrichtung,
- Fig. 3: in einer schematischen Ansicht, vergrößert, den Wirkbereich eines zweiten Ausführungsbeispiels einer erfindungsgemäßen Vorrichtung,
- Fig. 4: in einer schematischen Ansicht, vergrößert, den Wirkbereich eines dritten Ausführungsbeispiels einer erfindungsgemäßen Vorrichtung und
- Fig. 5: einer schematischen Ansicht, vergrößert, den Wirkbereich eines vierten Ausführungsbeispiels einer erfindungsgemäßen Vorrichtung.

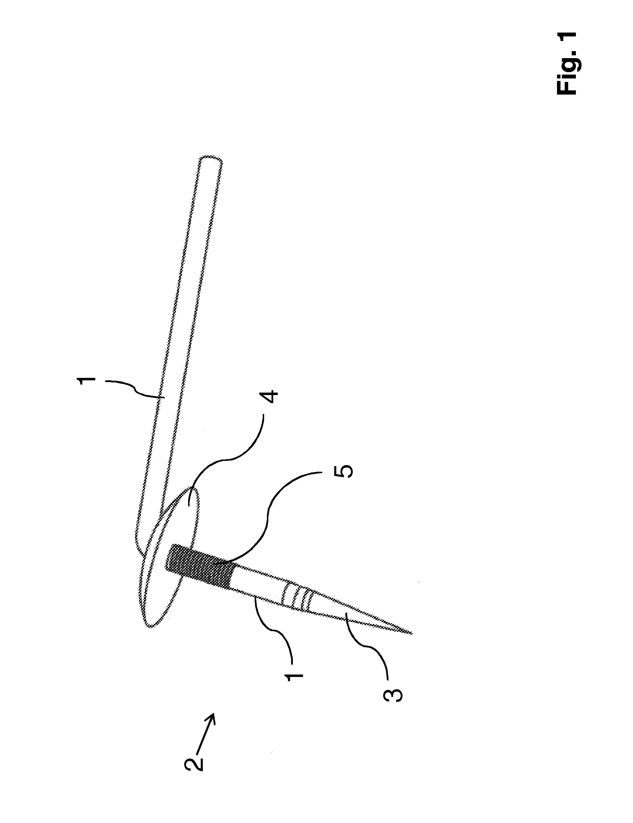

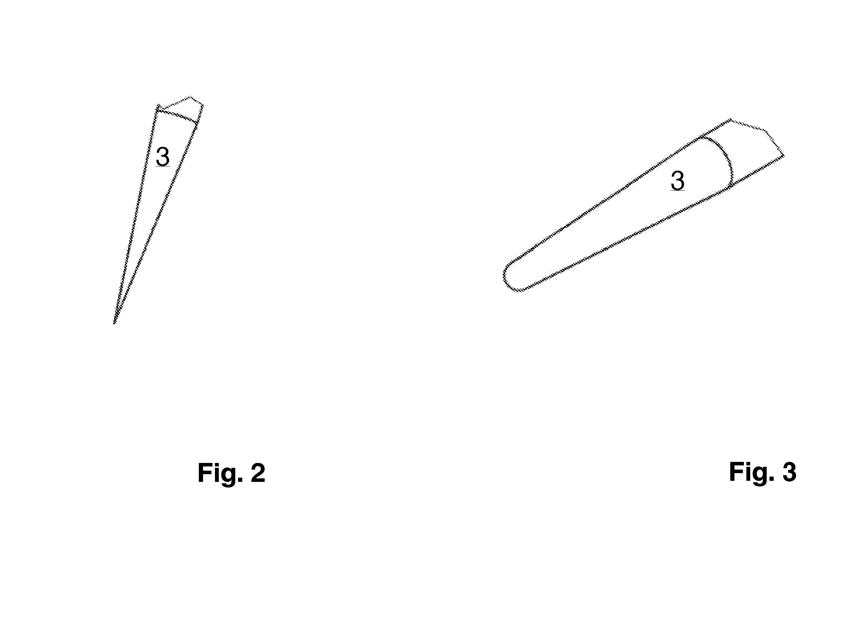

Fig. 1 zeigt den Lichtleiter 1 einer erfindungsgemäßen Vorrichtung zum Einleiten von Licht in ein menschliches oder tierisches Auge. Der Lichtleiter 1 umfasst einen nicht dargestellten Anschlussbereich zum Ankoppeln an eine Lichtquelle sowie ein freies Ende 2. Am freien Ende 2 ist ein Wirkbereich 3 zum Abstrahlen von Licht vorgesehen. Im hier dargestellten Ausführungsbeispiel weist der Wirkbereich 3 eine spitze Form auf, so dass die Sklera leicht mit dem Wirkbereich 3 durchstochen werden kann. In Fig. 2 ist der Wirkbereich 3 vergrößert dargestellt.

Fig. 1 ist weiterhin entnehmbar, dass das freie Ende 2 durch den Anschlusskörper 4 begrenzt ist. Befindet sich das freie Ende 2 im Augeninnern, so sitzt der Anschlusskörper 4 eng an der Oberfläche des Auges an. Um ein ungewolltes Herausstreifen des freien Endes 2 zu verhindern, ist der Anschlusskörper 4 flach ausgebildet.

Direkt am Anschlusskörper 4 weist das freie Ende 2 einen Fixierungsbereich 5 auf. Dieser dient dazu, das freie Ende 2 sicher im Auge zu fixieren. Dazu ist die Oberfläche im Fixierungsbereich 5 mit Riefen versehen.

Fig. 3 zeigt ein weiteres Ausführungsbeispiel des Wirkbereichs 3 einer erfindungsgemäßen Vorrichtung. Der Wirkbereich 3 ist an seinem Ende leicht gerundet ausgebildet. Hierdurch ist insbesondere im Endbereich eine größere Fläche zum Abstrahlen von Licht gegeben.

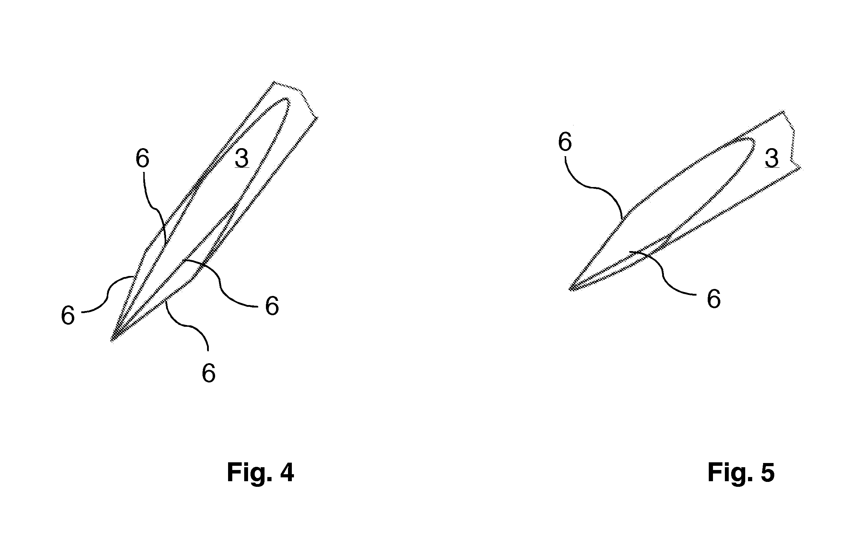

In Fig. 4 ist eine weitere Ausführungsform eines Wirkbereichs 3 gezeigt. Dieser weist scharfe Schneidkanten 6 auf. Zwischen den Schneidkanten 6 sind plane Flächen vorgesehen, wodurch das Licht auf definierte Weise abgestrahlt werden kann. Durch die Vielzahl von Schneidkanten 6 kann die Sklera in besonders einfacher Weise durchstochen werden.

Ein weiteres Ausführungsbeispiel des Wirkbereichs 3 einer erfindungsgemäßen Vorrichtung ist in Fig. 5 dargestellt. Der Wirkbereich 3 weist scharfe Schneidkanten 6 auf, die auf einer Seite eine plane Fläche und auf der gegenüberliegenden Seite eine gewölbte Fläche definieren.

In Bezug auf Merkmale, die sich den Figuren nicht entnehmen lassen, sei zur Vermeidung von Wiederholungen auf den allgemeinen Teil der Beschreibung verwiesen.

Schließlich sei ausdrücklich darauf hingewiesen, dass die voranstehend beschriebenen Ausführungsbeispiele der erfindungsgemäßen Vorrichtung zum Einleiten von Licht in ein menschliches oder tierisches Auge lediglich zur Erörterung der beanspruchten Lehre dienen, diese jedoch nicht auf die Ausführungsbeispiele einschränken.

### Bezugszeichenliste

- 1: Lichtleiter
- 2: freies Ende
- 3: Wirkbereich
- 4: Anschlusskörper
- 5: Fixierungsbereich
- 6: Schneidkante

## Patentansprüche

1. Vorrichtung zum Einleiten von Licht in ein menschliches oder tierisches Auge, mit einem Lichtleiter (1), der einen Anschlussbereich zum Ankoppeln an eine Lichtquelle und einen am freien Ende (2) ausgebildeten Wirkbereich (3) zum Abstrahlen von Licht aufweist,
**dadurch gekennzeichnet, dass** der Wirkbereich (3) zumindest teilweise als Stich- und/oder Schneidwerkzeug ausgebildet ist, nämlich scharfe Schneidkanten (6) und/oder Vorsprünge aufweist, um einen Schnitt beliebiger Länge anzubringen, so dass das freie Ende (2) ohne weitere Hilfsmittel in das Augeninnere einbringbar ist und dass das freie Ende (2) durch einen Anschlusskörper (4) begrenzt ist, wobei der Anschlusskörper (4) flach ausgebildet ist.

2. Vorrichtung nach Anspruch 1, **dadurch gekennzeichnet, dass** der Wirkbereich (3) spitz und/oder zumindest bereichsweise leicht gerundet ausgebildet ist.

3. Vorrichtung nach Anspruch 1 oder 2, **dadurch gekennzeichnet, dass** der Wirkbereich (3) einen Gewindegang oder eine gewendelte Korkenzieherform aufweist.

4. Vorrichtung nach einem der Ansprüche 1 bis 3, **dadurch gekennzeichnet, dass** der Wirkbereich (3) zumindest teilweise beschichtet oder ummantelt ist, um auf den Abstrahlwinkel des Lichts Einfluss zu nehmen.

5. Vorrichtung nach einem der Ansprüche 1 bis 4, **dadurch gekennzeichnet, dass** der Wirkbereich (3) eine derartige Geometrie, vorzugsweise in Form einer Linse, aufweist, dass das Licht auf einen Punkt fokussiert, in Form einer Spot-Beleuchtung abstrahlbar ist.

6. Vorrichtung nach einem der Ansprüche 1 bis 4, **dadurch gekennzeichnet, dass** der Wirkbereich (3) eine derartige Geometrie, vorzugsweise in Form einer Linse, aufweist, dass das Licht in einem weiten Winkel abstrahlbar ist.

7. Vorrichtung nach einem der Ansprüche 1 bis 6, **dadurch gekennzeichnet, dass** der Anschlusskörper (4) scheibenartig, tellerartig oder knopfartig ausgebildet ist.

8. Vorrichtung nach Anspruch 7, **dadurch gekennzeichnet, dass** der Anschlusskörper (4) als integraler Bestandteil des Lichtleiters (1) vorgesehen ist.

9. Vorrichtung nach Anspruch 7, **dadurch gekennzeichnet, dass** der Anschlusskörper (4) an den Lichtleiter (1) angeklemmt, angepresst oder angeklebt ist.

10. Vorrichtung nach einem der Ansprüche 1 bis 9, **dadurch gekennzeichnet, dass** der Anschlusskörper (4) zumindest bereichsweise oder insgesamt durchsichtig ausgeführt ist.

11. Vorrichtung nach einem der Ansprüche 1 bis 10, **dadurch gekennzeichnet, dass** das freie Ende (2) zumindest in einem an den Anschlusskörper (4) angrenzenden Fixierungsbereich (5) eine raue, genoppte oder im wesentlichen quer oder schräg zur Längsachse gewellte oder geriefte Oberfläche aufweist, oder dass zumindest der Fixierungsbereich (5) durch ein Außengewinde des freien Endes (2) gebildet ist.

12. Vorrichtung nach einem der Ansprüche 1 bis 11, **dadurch gekennzeichnet, dass** der Lichtleiter (1) aus Glasfaser, vorzugsweise Mineralglas oder Kieselglas, gefertigt ist.

13. Vorrichtung nach einem der Ansprüche 1 bis 11, **dadurch gekennzeichnet, dass** der Lichtleiter (1) aus polymeren optischen Fasern, vorzugsweise Polymethylmethacrylat (PMMA), gefertigt ist.

## Claims

1. Device for introducing light into a human or animal eye, having a light guide (1), which has a connection region for coupling to a light source and an active region (3) which is formed at the free end (2) for emitting light,
**characterised in that** the active region (3) is constructed at least partially as a stabbing and/or cutting tool, that is to say, has sharp cutting edges (6) and/or projections in order to apply a cut of any length so that the free end (2) can be introduced into the inner side of the eye without additional auxiliary means and **in that** the free end (2) is delimited by a connection member (4), the connection member (4) being constructed in a planar manner.

2. Device according to claim 1, **characterised in that** the active region (3) is constructed in a pointed and/or at least partially slightly rounded manner.

3. Device according to claim 1 or claim 2, **characterised in that** the active region (3) has a thread or a coiled corkscrew-like form.

4. Device according to any one of claims 1 to 3, **characterised in that** the active region (3) is at least partially coated or covered in order to influence the emission angle of the light.

5. Device according to any one of claims 1 to 4, **characterised in that** the active region (3) has such a geometry, preferably in the form of a lens, that the light is focused on a location, in the form of a spotlight.

6. Device according to any one of claims 1 to 4, **characterised in that** the active region (3) has such a geometry, preferably in the form of a lens, that the light can be emitted at a wide angle.

7. Device according to any one of claims 1 to 6, **characterised in that** the connection member (4) is constructed in a disc-like, plate-like or button-like manner.

8. Device according to claim 7, **characterised in that** the connection member (4) is provided as an integral component of the light guide (1).

9. Device according to claim 7, **characterised in that** the connection member (4) is clamped, pressed or adhesively bonded to the light guide (1).

10. Device according to any one of claims 1 to 9, **characterised in that** the connection member (4) is constructed so as to be at least partially or completely transparent.

11. Device according to any one of claims 1 to 10, **characterised in that** the free end (2) has at least in a fixing region (5) adjacent to the connection member (4) a surface which is rough, studded or undulating or grooved substantially transversely or obliquely relative to the longitudinal axis, or **in that** at least the fixing region (5) is formed by means of an outer thread of the free end (2).

12. Device according to any one of claims 1 to 11, **characterised in that** the light guide (1) is produced from glass fibre, preferably mineral glass or silica glass.

13. Device according to any one of claims 1 to 11, **characterised in that** the light guide (1) is produced from polymer light guides, preferably polymethylmethacrylate (PMMA).

## Revendications

1. Dispositif permettant d'injecter de la lumière dans un oeil humain ou animal, comportant un conducteur de lumière (1), qui comporte une zone de raccordement, destinée à être raccordée à une source lumineuse, et une zone active (3), réalisée au niveau de l'extrémité libre (2) et destinée à émettre de la lumière,
**caractérisé en ce que** la zone active (3) est réalisée au moins en partie sous forme d'outil de piqûre ou de coupe, c'est-à-dire qu'elle comporte des arêtes de coupe (6) et/ou saillies affûtées pour effectuer une coupe de longueur quelconque, de telle sorte que l'extrémité libre (2) peut être amenée sans autre aide à l'intérieur de l'oeil, et **en ce que** l'extrémité libre (2) est délimitée par un corps de raccordement (4), ledit corps de raccordement (4) étant plat.

2. Dispositif selon la revendication 1, **caractérisé en ce que** la zone active (3) est pointue et/ou légèrement arrondie au moins par zones.

3. Dispositif selon la revendication 1 ou 2, **caractérisé en ce que** la zone active (3) comporte un pas de vis ou une forme de tirebouchon en spirale.

4. Dispositif selon l'une quelconque des revendications 1 à 3, **caractérisé en ce que** la zone active (3) est au moins partiellement revêtue ou enveloppée pour pouvoir influer sur l'angle d'émission de la lumière.

5. Dispositif selon l'une quelconque des revendications 1 à 4, **caractérisé en ce que** la zone active (3) possède une géométrie, de préférence en forme de lentille, configurée de telle sorte que la lumière est focalisée en un point, peut être émise sous la forme d'un spot d'éclairage.

6. Dispositif selon l'une quelconque des revendications 1 à 4, **caractérisé en ce que** la zone active (3) possède une géométrie, de préférence en forme de lentille, configurée de telle sorte que la lumière peut être émise sur un large angle.

7. Dispositif selon l'une quelconque des revendications 1 à 6, **caractérisé en ce que** le corps de raccordement (4) est réalisé en forme de disque, de plateau ou de bouton.

8. Dispositif selon la revendication 7, **caractérisé en ce que** le corps de raccordement (4) est prévu sous la forme d'une partie intégrante du conducteur de lumière (1).

9. Dispositif selon la revendication 7, **caractérisé en ce que** le corps de raccordement (4) est serré, pressé ou collé contre le conducteur de lumière (1).

10. Dispositif selon l'une quelconque des revendications 1 à 9, **caractérisé en ce que** le corps de raccordement (4) est réalisé sous forme transparente au moins par zones ou en totalité.

11. Dispositif selon l'une quelconque des revendications 1 à 10, **caractérisé en ce que** l'extrémité libre (2) comporte, au moins dans une zone de fixation (5) adjacente au corps de raccordement (4), une surface rugueuse, à aspérités ou ondulée ou rainurée sensiblement transversalement à l'axe longitudinal, ou **en ce qu'**au moins la zone de fixation (5) est formée par un filetage extérieur de l'extrémité libre (2).

12. Dispositif selon l'une quelconque des revendications 1 à 11, **caractérisé en ce que** le conducteur de lumière (1) est réalisé en fibres de verre, de préférence du verre minéral ou du verre de quartz.

13. Dispositif selon l'une quelconque des revendications 1 à 11, **caractérisé en ce que** le conducteur de lumière (1) est réalisé en fibres optiques polymères, de préférence en polyméthacrylate de méthyle (PMMA).
